(19) Europäisches Patentamt
European Patent Office
Office européen des brevets

(11) Veröffentlichungsnummer: **0 264 577**
**A1**

(12) # EUROPÄISCHE PATENTANMELDUNG

(21) Anmeldenummer: **87112287.5**

(51) Int. Cl.4: **C07D 233/90** , A01N 43/50

(22) Anmeldetag: **25.08.87**

Patentanspruch für folgende Vertragsstaat: ES.

(30) Priorität: **27.08.86 DE 3629064**

(43) Veröffentlichungstag der Anmeldung: **27.04.88 Patentblatt 88/17**

(84) Benannte Vertragsstaaten: **AT BE CH DE ES FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Schmierer, Roland, Dr.**
**An der Weinleite 5a**
**D-8901 Todtenweis(DE)**
Erfinder: **Mildenberger, Hilmar, Dr.**
**Fasanenstrasse 24**
**D-6233 Kelkheim Taunus(DE)**
Erfinder: **Bürstell, Helmut, Dr.**
**Am Hohlacker 65**
**D-6000 Frankfurt am Main 50(DE)**

(54) **2,3,6-Substituierte Phenylimidazolderivate, Verfahren zu ihrer Herstellung und ihre Verwendung als Wachstumsregulatoren.**

(57) Die Verbindungen der Formel I oder deren Salze

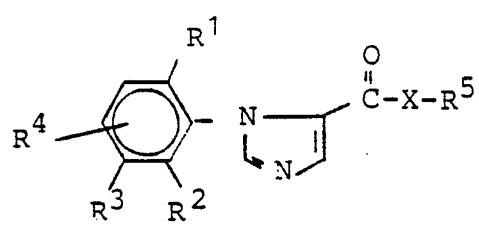

(I),

worin
$R^1$ und $R^2$ unabhängig voneinander ($C_1$-$C_4$)-Alkyl;
$R^3$ Halogen, insbesondere Cl oder Br
$R^4$ Wasserstoff, Cl, Br oder Methyl,
X O, S oder N-$R^5$
$R^5$ Wasserstoff, ($C_2$-$C_6$)-Alkenyl, ($C_1$-$C_6$)-Alkyl, wobei die Alkylgruppe bis zu zweifch durch ($C_1$-$C_6$)-Alkoxy, ($C_1$-$C_3$)-Dialkylamino oder bis zu sechsfach durch Halogen substituiert sein kann, bedeutet, besitzen hervorragende pflanzenwachstumsregulierende Wirkung in wichtigen Kulturen.

EP 0 264 577 A1

## 2,3,6-Substituierte Phenylimidazolderivate, Verfahren zu ihrer Herstellung und ihre Verwendung als Wachstumsregulatoren

1-Phenyl-imidazol-5-carbonsäurederivate sowie deren Verwendung als Fungizide, Herbizide und Pflanzenwachstumsregulatoren sind aus DE-A 32 17 094 bekannt.

Es wurde nun überraschenderweise gefunden, daß ausgewählte in 3-Stellung des Phenylrings halogensubstituierte Imidazolverbindungen besonders intensive wachstumsregulierende Wirkung besitzen.

Gegenstand der vorliegenden Erfindung sind daher die Verbindungen der Formel I

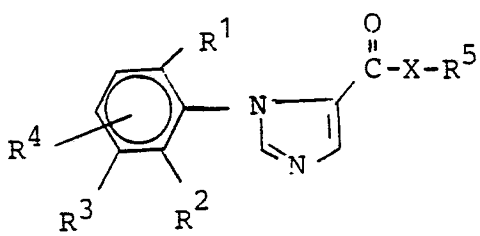

(I),

worin

$R^1$ und $R^2$ unabhängig voneinander $(C_1-C_4)$-Alkyl;
$R^3$ Halogen, insbesondere Cl oder Br,
$R^4$ Wasserstoff, Cl, Br oder Methyl,
X O, S oder N-$R^5$
$R^5$ Wasserstoff, $(C_2-C_6)$-Alkenyl, $(C_1-C_6)$-Alkyl, wobei die Alkylgruppe bis zu zweifach durch $(C_1-C_6)$-Alkoxy, $(C_1-C_3)$-Dialkylamino oder bis zu sechsfach durch Halogen substituiert sein kann, bedeutet,
sowie deren für landwirtschaftliche Zwecke verträglichen Salze und Quarternisierungsprodukte.

Als halogeniertes $(C_1-C_6)$Alkyl für $R^5$ kommen insbesondere in Betracht die Reste Hexafluoropropyl, 2,2,3,3-Tetrafluoropropyl; 2,2,2-Trifluorethyl; 2,2,2-Trichlorethyl; 2,2,3,4,4,4-Hexafluorbutyl.

Die Salzbildung bzw. Quaternisierung erfolgt entweder an der -COOH oder(C=O)-SH-Gruppe (X = O, S; $R^5$= H) oder am basischen Stickstoffatom des Imidazolrings. Die Salzbildung oder Quaternisierung am Imidazolring ist nicht möglich, wenn $R^5$ ein Kation enthält, Unter Halogen ist F, Cl, Br oder J, insbesondere F, Cl oder Br zu verstehen.

Für die Salze geeignet sind alle anorganischen oder organischen Säuren, die aufgrund ihres pKs-Wertes zur Salzbildung befähigt sind, z.B. Halogenwasserstoffsäuren, Salpetersäure, Schwefelsäure, Phosphorsäure, Phosphonsäuren, Sulfonsäuren, Halogenessigsäuren oder Oxalsäure.

Als Kationen, die für die Landwirtschaft einsetzbar sind, kommen Metallkationen z.B. Alkali-oder Erdalkalikationen wie Na, K, Mg oder Ammonium oder organische Kationen, wie beispielsweise durch organische Reste substituierte Ammonium-, Phosphonium-, Sulfonium-oder Sulfoxonium-Ionen oder gegebenenfalls substituiertes Hydrazonium, Hydroxylammonium, Guanidinium oder Aminoguanidinium in Betracht.

Organisch substituiertes Ammonium bedeutet primäres, sekundäres, tertiäres, quartäres, aliphatisches, aromatisches oder heteroaromatisches Ammonium, das 1 bis drei N-Atome enthalten kann. Die Stickstoffatome des Amins können hierbei auch Teil eines cyclischen Systems sein. Als Beispiele für solche Ammoniumsalze seien genannt: Mono-, Di-, Tri-, Tetra[$(C_1-C_6)$Alkyl]ammonium wie Isopropylammonium, Butylammonium, Stearylammonium, Triethylammonium, Mono-, Di-, Tri-[$(C_1-C_4)$alkoxy$(C_1-C_4)$alkyl]-ammonium oder Mono-, Di-, Tri-[$(C_1-C_6)$-alkanol]-ammonium wie Methoxyethylammonium, Methoxypropylammonium, Triethanolammonium, Tripropanolammonium, oder Ammoniumverbindungen mit gemischten Resten wie tert.-Butyldiethanolammonium, Triethylenbenzylammonium, Hydroxyethyltrimethylammonium, Chlorethyltrimethylammonium; oder Allylammonium, Diallyl ammonium, Cyclohexylammonium, Menthylammonium, Aminoethylammonium, Ethylendiammonium, Benzhydrylammonium, Pyrrolidinium, Morpholinium, 3-Pyridylammonium, Piperidinium oder Piperazinium, oder ein von einer Aminosäure oder deren Ester abgeleitetes Ammonium wie $^{\oplus}NH_3-CH_2-COOCH_3$.

Entsprechende Reste wie bei Ammonium können in den anderen obengenannten organischen Kationen vorliegen.

Entsprechende Reste wie bei Ammonium können in den anderen obengenannten organischen Kationen vorliegen.

Gegenstand der Erfindung sind ferner Verfahren zur Herstellung der Verbindungen der Formel I und ihrer Salze oder Quaternisierungsprodukte, dadurch gekennzeichnet, daß man

a) einen Bisformylester der Formeln (IIa) oder (IIb)

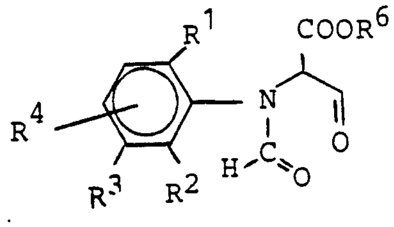

(IIa)

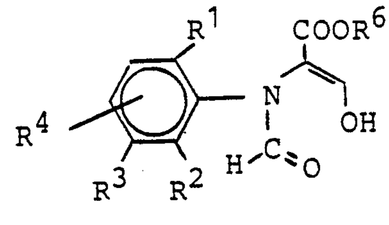

(IIb)

wobei $R^6$ = ($C_1$-$C_{12}$)-Alkyl bedeutet,
in Gegenwart von Ammoniak oder einer Ammoniak freisetzenden Verbindung erhitzt, oder

b) ein Aminophenylderivat der Formel III

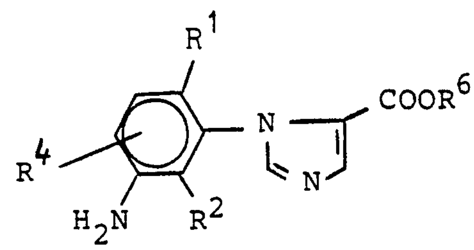

(III)

diazotiert und anschließend mit einem Halogenid gemäß der Sandmeyer-Reaktion umsetzt, oder

c) für den Fall, daß $R^3$ für Chlor oder Brom steht, ein Phenylimidazolderivat der Formel IV

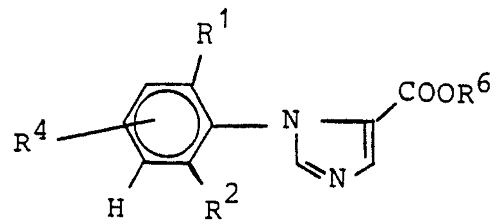

(IV)

bromiert oder chloriert oder die unter a), b) oder c) erhaltenen Verbindungen derivatisiert.

Bei der Derivatisierung wird der Rest -$COOR^6$ in bekannter Weise modifiziert z.B. durch Verseifung, Veresterung, Umesterung, Amidierung oder Salzbildung, wie dies z.B. in den Patentanmeldungen DE-A 34 44 918 und DE-A 34 42 690 beschrieben ist, oder es erfolgt Salzbildung oder Quaternisierung am basischen Stickstoffatom des Imidazolringes.

Zu a) Die Formylverbindungen IIa/IIb lassen sich nach bekannten Verfahren, wie sie z.B. in der DE-A 32 17 094 beschrieben sind, aus den entsprechenden Anilinen herstellen. Die Cyclisierung der Formylverbindungen in Gegenwart von Ammoniak bzw. in der Hitze Ammoniak bildenden Substanzen wie z.B. Ammoniumacetat, Ammoniumbenzoat oder Formamid erfolgt, ggf. in Gegenwart hochsiedender Lösungsmittel wie z.B. Xylol oder Formamid bei Temperaturen von 100 bis 200°C.

Zu b) Die Aminoverbindungen sind aus den bekannten Imidazolen der Formel IV (DE-A 32 17 094) durch Nitrierung und Reduktion zugänglich.
Die Sandmeyer-Reaktion läßt sich unter üblichen Reak tionsbedingungen (vgl. z.B. Organikum, VEB Deutscher Verlag der Wissenschaften Berlin 1973, S. 590 ff) in guter Ausbeute durchführen.

Zu c) Die Halogenierung läßt sich ebenfalls nach üblichen Verfahren (vgl. z.B. Organikum s.o. S. 342 ff) aus den bekannten Verbindungen der Formel IV durchführen.
Die Salze der Verbindungen der Formel (I) können in einfacher Weise nach üblichen Salzbildungsmethoden, z.B. durch Lösen einer Verbindung der Formel (I) in einem geeigneten organischen Lösemittel und Hinzufügen der Säure erhalten werden und in bekannter Weise, z.B. durch Abfiltrieren, isoliert und gegebenenfalls durch Waschen mit einem inerten organischen Lösemittel gereinigt werden.

Mit den erfindungsgemäßen Verbindungen sind typische wachstumsregulierende Effekte erzielbar, die - verglichen mit den aus der DE-A 32 17 094 bekannten Verbindungen -überraschenderweise bereits bei sehr niedrigen Dosierungen erzielt werden können.

Die erfindungsgemäßen Verbindungen greifen regulierend in den pflanzeneigenen Stoffwechsel ein und können damit zur gezielten Beeinflussung von Pflanzeninhaltsstoffen sowie zur Ernteerleichterung wie zum Auslösen von Desikkation und Wuchsstauchung eingesetzt werden. Des weiteren eignen sie sich zur generellen Steuerung und Hemmung von unerwünschtem vegetativen Wachstum, ohne dabei die Pflanzen abzutöten. Eine Hemmung des vegetativen Wachstums spielt bei vielen mono-und dikotylen Kulturen eine große Rolle, da das Lagern hierdurch verringert oder völlig verhindert werden kann. Besonders hervorzuheben ist die wachstumsregulatorische Wirksamkeit der Verbindungen als Wuchshemmer in Getreide, Mais, Soja, Tabak, Baumwolle, Ackerbohne, Raps, Reis, Sonnenblume, Rasen sowie ihre Fähigkeiten, den Gehalt an erwünschten Inhaltsstoffen wie Kohlehydraten (z.B. Zuckerrohr oder Hirsekulturen) und Protein bei Nutzpflanzen zu erhöhen.

Die erfindungsgemäßen Verbindungen lassen sich bei ihrem praktischen Einsatz gegebenenfalls auch vorteilhaft mit bekannten Wachstumsregulatoren kombinieren. Solche bekannten Wachstumsregulatoren sind die Verbindungen der Formel (V)

$R\text{-}CH_2\text{-}CH_2\text{-}N^+(CH_3)_3Cl^-$ (V)

worin R = OH oder Cl bedeutet (common name Chlormequat für R = Cl), ferner N,N-Dimethylpiperidiniumchlorid (VI; common name: Mepiquatchlorid), α-Cyclopropyl-4-methoxy-α-(pyrimidin-5-yl)benzylalkohol (VII; common name: Ancymidol) (3aα, 4β, 4aα, 6aα, 7β, 7aα)-1-(4-chlorophenyl)-3a, 4, 4a, 6a, 7, 7a-hexahydro-4,7-methano-1H-[1,2]diazeto[3,4-f] benzotriazol (VIII; common name: Tetcylacis), Bernsteinsäure-mono-2,2-dimethylhydrazid (IX, common name: Diaminoazide), 6-Hydroxy-2H-pyridazin-3-on (X, Maleinsäureanhydrid), 2-Chlor-9-hydroxy-9H-fluoren-9-carbonsäure (XI, Chlorflurenol), 5'-(Triflzormethansulfonamido)acetat-2',4'-xylidid (Mefluidid), 2-Chlorethylphosphonsäure (XIII, Ethephon).

Die wachstumsregulatorischen Wirkungen der Verbindungen der Formeln (V) bis (XIII) sind beschrieben in Plant Growth Regulator Handbook of the Plant Growth Regulator Working Group 2d. Ed. 1981.

Anstelle der Verbindungen der Formeln (V) und (VI) können prinzipiell auch vergleichbare Salze, die anstelle des Chloridions ein anderes übliches Anion wie Bromid, Nitrat oder 1/2 Sulfat enthalten, eingesetzt werden.

Die Kombinationen können sowohl als Mischformulierungen der Komponenten vorliegen, die dann in üblicher Weise mit Wasser verdünnt zur Anwendung gebracht werden; oder sie können als sogenannte Tankmischungen durch gemeinsame Verdünnung der getrennt formulierten Komponenten mit Wasser hergestellt werden; es besteht auch die Möglichkeit, die Komponenten nacheinander zur Anwendung zu bringen, d.h. es werden dann die Komponenten in Einzelformulierungen appliziert.

Die Verbindungen der allgemeinen Formel (I) können auch mit natürlichen oder pflanzlichen Hormonen wie Auxinen oder Cytokinen kombiniert werden.

Eine weitere Lösung der gestellten Aufgabe sind auch das Pflanzenwachstum regulierende Mittel, die sich durch einen wirksammen Gehalt mindestens einer der erfindungsgemäßen Verbindungen auszeichnen.

Die erfindungsgemäßen Verbindungen können, gegebenenfalls im Gemisch mit weiteren Wirkkomponenten, als Spritzpulver, emulgierbare Konzentrate, versprühbare Lösungen, Stäubemittel, Dispersionen, Granulate oder Mikrogranulate in den üblichen Zubereitungen angewendet werden.

Spritzpulver sind in Wasser gleichmäßig dispergierbare Präparate, die neben dem (den) Wirkstoff(en) außer gegebenenfalls einem Verdünnungs-oder Inertstoff oder Netzmittel wie polyoxethylierte Fettalkohole, Alkyl-oder Alkylphenylsulfonate und/oder Dispergierhilfsmittel wie ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen der Wirkstoffe in einem inerten organischen Lösemittel wie Butanol, Cyclohexanon, Dimethylformamid, Xylol oder auch höhersiedenden Aromaten oder aliphatischen oder cycloaliphatischen Kohlenwasserstoffen unter Zusatz von einem oder mehreren Emulgatoren hergestellt werden. Bei flüssigen Wirkstoffen kann der Lösemittelanteil auch ganz oder teilweise entfallen. Als Emulgatoren können beispielsweise verwendet werden: Alkyl-arylsulfonsaure Calciumsalze wie

Ca-dodecylbenzolsulfonat oder nichtionische Emulgatoren wie Fettsäurepolyglykolester, Alkylarylpolyglykolether, Fettalkoholpolyglykolether, PropylenoxidEthylenoxid-Kondensationsprodukte, Fettalkohol-Propylenoxid-Kondensationsprodukte, Alkylpolyglykolether, Sorbitanfettsäureester, Polyoxethylensorbitansäureester oder Polyoxethylensorbitester.

Stäubemittel kann man durch Vermahlen der Wirkstoffe mit feinverteilten, festen Stoffen, z.B. Talkum, natürlichen Tonen wie Kaolin, Bentonit, Pyrophillit oder Diatomeenerde erhalten. Granulate können entweder durch Verdüsen der Wirkstoffe auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration etwa 5 bis 90 Gew.-%, der Rest zu 100 Gew.-% besteht aus üblichen Formulierungsbestandteilen. Bei emulgierbaren Konzentraten kann die Wirkstoffkonzentration etwa 3 bis 80 Gew.-% betragen. Staubförmige Formulierungen enthalten meistens 0,025 bis 20 Gew.-% an Wirkstoff(en), versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden. Daneben enthalten die genannten Wirkstofformulierungen gegebenen falls die jeweils üblichen Haft-, Netz-, Dispergier-, Emulgier-, Penetrations-, Lösemittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Die Aufwandmenge der erfindungsgemäßen Verbindungen variiert im allgemeinen zwischen 0,02 bis 2,5 kg Wirksubstanz pro Hektar, vorzugsweise 0,05 bis 1,5 kg/ha.

Die nachfolgenden Beispiele dienen zur Erläuterung der Erfindung.

## Formulierungsbeispiele

### Beispiel 1

Ein Stäubemittel wird erhalten, indem man a) 10 GT (Gewichtsteile) Wirkstoff mit 90 GT Talkum oder einem anderen Inertstoff mischt und in einer Schlagmühle zerkleinert, oder indem man b) 60 GT Wirkstoff, 35 GT Talkum und 5 GT Haftmittel (z.B. ein Polysaccharid wie Rhodopol der Rhone-Poulenc S.A.) in der gleichen Weise homogenisiert.

### Beispiel 2

Ein in Wasser leicht dispergierbares, benetzbares Pulver wird erhalten, indem man 25 GT Wirkstoff, 64 GT kaolinhaltigen Quarz als Inertstoff, 10 GT ligninsulfonsaures Kalium und 1 GT oleoylmethyltaurinsaures Natrium als Netz-und Dispergierhilfsmittel mischt und in einer Stiftmühle mahlt. Eine Formulierung mit 5% Wirkstoffgehalt kann wie folgt zusammengesetzt sein: 5% Wirkstoff. 6% eines sulfonierten Naphthalinformaldehydkondensats (z.B. ®Dispersogen A der Hoechst AG), 2% eines Na-Salzes einer Alkylnaphthalinsulfonsäure (z.B. ®Leonil DB der Hoechst AG), 5% eines Gemisches aus Polypropylenglykol und $SiO_2$ (z.B. ®Acrotin 341 der Hoechst AG), 25% eines Silikats (z.B. Sipernat der Degussa AG) und 57% Kaolin Typ 1777.

### Beispiel 3

Ein in Wasser leicht dispergierbares Dispersionskonzentrat wird erhalten, indem man 20 GT Wirkstoff mit 6 GT eines Alkylphenolpolyglykolethers (z.B. ®Triton X 207 von Rohm and Haas Co.), 3 GT Isotridecanolpolyglykolether (8 Ethylenoxid-Einheiten) und 71 GT paraffinischem Mineralöl (Siedebereich ca. 255 bis über 377°C) mischt und in einer Reibkugelmühle auf eine Feinheit von unter 5µm vermahlt.

Beispiel 4

Ein emulgierbares Konzentrat wird erhalten aus 15 GT Wirkstoff(e), 75 GT Cyclohexanol als Lösemittel und 10 GT oxethyliertem Nonylphenol (10 Ethylenoxid-Einheiten)als Emulgatoren.

Chemische Beispiele

Beispiel 1

Ethyl-1-(3-Chlor-6-ethyl-2-methylphenyl)-imidazol-5-carboxylat

19,2 g (0,068 Mol)2-(3-Chlor-6-ethyl-2-methyl-N-formylanilino)-3-hydroxy-acrylsäureethylester wurden mit 12,3 g (0,088 Mol) Ammoniumbenzoat in 100 ml Xylol unter Abdestillieren des Xylol-Wassergemischs bis auf 153 °C Innentemperatur erhitzt. Man ließ abkühlen, nahm in Toluol auf, wusch die organische Phase 2 mal mit 2-N-Natronlauge und 2mal mit Wasser, trocknete über Natriumsulfat und dampfte ein. Man erheilt 15, 2 g (85 % der Theorie) Ethyl-1-(3-chlor-6-ethyl-2-methylphenyl) imidazol-5-carboxylat als hellbraunes Öl. Die Identifizierung erfolgte $^1$H-NMR spektroskopisch.

Beispiel 2

Ethyl-1-(3-brom-2,6-diethylphenyl)-imidazol-5-carboxylat

Zu einem Gemisch aus 30 ml konzentrierter Bromwasserstoffsäure, 30 ml Eisessig und 30 ml Wasser gab man 20 g (0,07 Mol) Ethyl-1-(3-amino-2,6-diethyl-phenyl) imidazol-5-carboxylat zu, kühlte auf 0 °C ab und tropfte bei dieser Temperatur eine Lösung von 12,0 g (0,17)Mol Natriumnitrit in 15 ml Wasser zu. Nach 30 Minuten bei 0 °C zersetzte man das überschüssige Nitrit mit Harnstoff, und tropfte die Lösung bei 0 °C zu einer Kupferbromidlösung (hergestellt aus 24,9 g Kupfersulfat, 15,5 g Natriumbromid, 6,3 g Natriumsulfit, 80 ml Wasser und 40 ml konzentrierter Bromwasserstoffsäure nach üblichen Verfahren) zu. Man ließ auf Raumtemperatur erwärmen, neutralisierte das Reaktionsgemisch, extrahierte das Produkt mit Methylenchlorid, wusch die organische Phase mit Wasser, trocknete über Natriumsulfat und dampfte ein. Nach chromatographischer Reinigung erhielt man 17,4 g (71 % der Theorie) Ethyl-1-(3-brom-2,6-diethylphenyl)-imidazol-5-carboxylat ein leicht hellgelbes öl. Die Identifizierung erfolgte $^1$H-NMR-spektroskopisch.

Beispiel 3

1-(3-Brom-2,6-diethylphenyl)-imidazol-5-carbonsäure

17,4 g des in Beispiel 2 beschriebenen Ethylesters wurden mit 150 ml 2-N-Natronlauge 1 Stunde auf 80 °C erhitzt. Nach dem Abkühlen wurde mit konzentrierter Salzsäure auf pH 3 angesäuert und der farblose Feststoff abgesaugt. Nach dem Trocknen erhält man 11,6 g (72 % der Theorie) 1-(3-Brom-2,6-diethylphenyl)-imidazol-5-carbonsäure vom Schmelzpunkt 224-7 °C.

Nach den vorstehend beschriebenen Verfahren lassen sich die folgenen Beispiele herstellen.
Stehen in der Tabelle 1 für Y andere Reste als $-COOCH_3$ und $-COOC_2H_5$ wurden diese nach gängigen, allgemein bekannten Verfahren, wie z.B. Verseifung (Beispiel 3), anschließende Herstellung des Säurechlorids und Umsetzung mit Alkoholen, Aminen, Thiolen; oder Umsetzung der Säure mit Basen zur Salzbildung, aus den entsprechenden Estern hergestellt.

Tabelle 1 Imidazolderivate

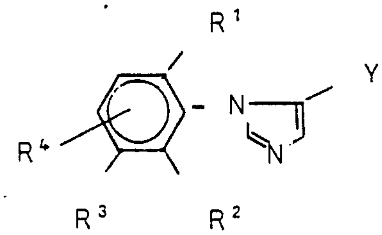

| Beisp.-Nr. | $R^1$ | $R^2$ | $R^3$ | $R^4$ | Y | Fp/Sdp. (°C) |
|---|---|---|---|---|---|---|
| 4 | $CH_3$ | $CH_3$ | Br | H | $-COOCH_3$ | 132-5 |
| 5 | " | " | " | " | $-COOH$ | 227-9 |
| 6 | " | " | " | " | $-COO^{\ominus} \cdot NH_4^{\oplus}$ | |
| 7 | " | " | " | " | $-C(=O)S-C_2H_5$ | |
| 8 | " | " | " | " | $-C(=O)S-CH_3$ | |
| 9 | " | $C_2H_5$ | Cl | " | $-COOH$ | 179-82 |
| 10 | " | " | " | " | $-COOK$ | |
| 11 | " | " | " | " | $-COO \cdot 1/2\ Mg$ | |
| 12 | " | " | " | " | $-COOCH_3$ | 101 |
| 13 | " | " | " | " | $-COOC_2H_5$ | 135-7/0.01 Torr |
| 14 | " | " | " | " | $-C(=O)NH_2$ | 168-72 |
| 15 | " | " | " | " | $-C(=O)i-NH-C_4H_9$ | |
| 16 | " | " | " | " | $-C(=O)-NHCH_3$ | 209-13 |
| 17 | " | " | " | " | $-C(=O)N(CH_3)_2$ | Öl |
| 18 | " | " | " | " | $-C(=O)NH-(CH_2)_3-OCH_3$ | |
| 19 | " | " | " | " | $-C(=O)N(CH_3)-CH(CH_3)_2$ | |
| 20 | " | " | " | " | $-COOCH_2-CCl_3$ | |
| 21 | " | " | " | " | $-C(=O)NH-CH_2-CH=CH_2$ | |
| 22 | " | " | " | " | $-COOCH_3$ (Hydrochlorid) | |
| 23 | $C_2H_5$ | $CH_3$ | Cl | " | $-COOH$ | 185-92 |
| 24 | " | " | " | " | $-COO^{-} \cdot [N(C_2H_5)_3]^{+}$ | |
| 25 | " | " | " | " | $-COO^{-} \cdot$ 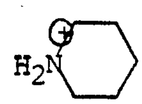 | |
| 26 | " | " | " | " | $-COONa$ | |

...

Fortsetzung Tabelle 1

| Beisp.-Nr. | $R^1$ | $R^2$ | $R^3$ | $R^4$ | Y | Fp/Sdp (°C) |
|---|---|---|---|---|---|---|
| 27 | $C_2H_5$ | $CH_3$ | Cl | H | $-COO^{\ominus} \cdot S^{\oplus}(CH_3)_3$ | |
| 28 | " | " | " | " | $-COOCH_3$ | 105-10 |
| 29 | " | " | " | " | $-COO-i-C_3H_7$ | Öl |
| 30 | " | " | " | " | $-COOC_6H_{13}$ | |
| 31 | " | " | " | " | $-COO-(CH_2)_2-O-C_2H_5$ | |
| 32 | " | " | " | " | $-COO-CH(CF_3)_2$ | |
| 33 | " | " | " | " | $-C(=O)NH_2$ | 170 |
| 34 | " | " | " | " | $-C(=O)N(-C_2H_5)_2$ | Öl |
| 35 | " | " | " | " | $-C(=O)N(-CH_3)-CH(CH_3)_2$ | |
| 36 | " | " | " | " | $-C(=O)N(CH_2-CH=CH_2)_2$ | |
| 37 | $C_2H_5$ | $C_2H_5$ | Cl | " | $-COOH$ | 210-4 (Zers.) |
| 38 | " | " | " | " | $-COONa$ | 144-8 |
| 39 | " | " | " | " | $-COOK$ | 210-14 |
| 40 | " | " | " | " | $-COO^{\ominus}[HN(-CH_2-CH_2-OH)_3]^{\oplus}$ | 89-94 |
| 41 | " | " | " | " | $-COO^{\ominus}[P^{\oplus}(CH_3)(Phenyl)_3]$ | |
| 42 | " | " | " | " | -COOH (Hydrochlorid) | |
| 43 | " | " | " | " | -COOH(2-Chlorethyl-phosphonat) | |
| 44 | " | " | " | " | -COOH (Sulfonat) | |
| 45 | " | " | " | " | $COOCH_3$ | Öl |
| 46 | " | " | " | " | $-COOC_2H_5$ | 153-7/0.01 Torr |
| 47 | " | " | " | " | $-COOCH_2-CF_3$ | |
| 48 | " | " | " | " | $-COOCH_2-CHF-CF_2-CF_3$ | |
| 49 | " | " | " | " | $-COOCH_2-CH_2-N(C_2H_5)_2$ | |
| 50 | " | " | " | " | $-COO-n-C_3H_7$ | Öl |
| 51 | " | " | " | " | $-COO-CH_2-CH=CH_2$ | Öl |
| 52 | " | " | " | " | $-COO-n-C_6H_{13}$ | |
| 53 | " | " | " | " | $-COO-i-C_3H_7$ | |
| 54 | " | " | " | " | $-C(=O)-NH_2$ | 188-93 |
| 55 | " | " | " | " | $-C(=O)-NH-CH_2-CH(OCH_3)_2$ | 110-4 |
| 56 | " | " | " | " | $-C(=O)-N(CH_3)[CH_2-CH(OC_2H_5)_2]$ | |
| 57 | " | " | " | " | $-C(=O)NH-i-C_3H_5$ | |
| 58 | " | " | " | " | $-C(=O)NH-CH_2-i-C_3H_7$ | |

...

Fortsetzung Tabelle 1

| Beisp.-Nr. | $R^1$ | $R^2$ | $R^3$ | $R^4$ | Y | Fp/Sdp (°C) |
|---|---|---|---|---|---|---|
| 59 | $C_2H_5$ | $C_2H_5$ | Cl | H | $-C(=O)NH-CH_3$ | 187-94 |
| 60 | " | " | " | " | $-C(=O)N(-CH_3)_2$ | Öl |
| 61 | " | " | " | " | $-C(=O)-SH$ | |
| 62 | " | " | " | " | $-C(=O)S\ C_2H_5$ | |
| 63 | " | " | " | 4-Br | $-COOH$ | |
| 64 | " | " | " | " | $-COOC_2H_5$ | 70-6 |
| 65 | " | " | " | " | $-COO-CH_3$ | |
| 66 | " | " | " | " | -COO 1/2 Ca | |
| 67 | " | " | " | 4-Br | $.C(=O)NH_2$ | |
| 68 | " | " | " | " | $-C(=O)NH-n-C_6H_{13}$ | |
| 69 | " | " | " | $4-CH_3$ | $-COOH$ | 213-5 |
| 70 | " | " | " | " | $-COO^{\ominus}$ 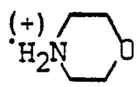 | Harz |
| 71 | " | " | " | " | $-COOCH_3$ | Öl |
| 72 | " | " | " | " | $-COOC_2H_5$ | Öl |
| 73 | " | " | " | " | $-COO(CH_2)_2-O-CH_3$ | |
| 74 | " | " | " | " | $-C(=O)NH_2$ | 163-5 |
| 75 | " | " | " | " | $-C(=O)NH_2$(Hydrochlorid) | |
| 76 | " | " | " | " | $-C(=O)-NH-C_4H_9$ | |
| 77 | " | " | " | 5-Cl | $-COOH$ | 197-202 |
| 78 | " | " | " | " | $-COO^{\ominus}\ [HN^{(+)}(C_2H_5)_2]$ | |
| 79 | " | " | " | " | $-COO^{\ominus}\ [Cl-CH_2-CH_2-N^{\oplus}(CH_3)_3]$ | |
| 80 | " | " | " | " | $-COOCH_3$ | 32-4 |
| 81 | " | " | " | " | $-COOC_2H_5$ | 132-36 |
| 82 | " | " | " | " | $-COO-CH(CH_3)C_6H_{13}$ | |
| 83 | " | " | " | " | $-C(=O)NH_2$ | 179-85 |
| 84 | " | " | " | " | $-C(=O)NH-CH(CH_3)_2$ | |
| 85 | " | " | " | " | $-C(=O)NH-i-C_5H_{11}$ | |
| 86 | " | " | " | " | $-C(=O)N(CH_3)CH_2\ CF_3$ | |
| 87 | " | " | F | H | $-COOH$ | |
| 88 | " | " | " | " | $-COOCH_3$ | |
| 89 | " | " | " | " | $-COOC_2H_5$ | |

...

Fortsetzung Tabelle1

| Beisp.-Nr. | $R^1$ | $R^2$ | $R^3$ | $R^4$ | Y | Fp/Sdp (°C) |
|---|---|---|---|---|---|---|
| 90 | $C_2H_5$ | $C_2H_5$ | F | H | $-C(=O)NH_2$ | |
| 91 | " | " | J | " | -COOH | 202-4 |
| 92 | " | " | " | " | $-COOC_2H_5$ | Öl |
| 93 | " | " | Cl | " | -COOH | |
| 94 | " | " | " | " | $-COOC_2H_5$ | |
| 95 | " | " | " | " | $-C(=O)NH_2$ | |
| 96 | " | " | " | $5-CH_3$ | -COOH | |
| 97 | " | " | " | " | $-COOC_2H_5$ | |
| 98 | " | " | " | " | $-C\ =O)NH_2$ | |
| 99 | " | " | " | " | $-C\ =O)NH-CH_3$ | |
| 100 | $CH(CH_3)_2$ | $CH(CH_3)_2$ | Cl | H | -COOH | |
| 101 | " | " | " | " | $-COO^{(-)}[H_3\overset{(+)}{N}-CH_2COOCH_3]$ | |
| 102 | " | " | " | " | $-COO^{(-)}$ 1/2 $Zn^{(+)}$ | |
| 103 | " | " | " | " | $-COOCH_3$ | |
| 104 | " | " | " | " | $-COO(CH_2)_2-O-C_4H_9$ | |
| 105 | " | " | " | " | $-COOCH(CF_3)_2$ | |
| 106 | " | " | " | " | $-C(=O)NH_2$ | |
| 107 | " | " | " | " | $-C(=O)N(CH_3)_2$ | |
| 108 | " | " | " | " | $-C(=O)NHCH_2-CH=CH_2$ | |
| 109 | " | " | " | " | $-C(=O)NHC_6H_{13}$ | |
| 110 | " | " | " | " | $-C(=O)NH(CH_2)_3-N(C_2H_5)_2$ | |
| 111 | $CH_3$ | $CH_3$ | Br | H | $-COOC_2H_5$ | 78-81 |
| 112 | " | " | " | " | $-COOC_3H_7$ | 100-5 |
| 113 | " | " | " | " | $-COOCH(CF_3)_2$ | 92-5 |
| 114 | " | " | " | " | $-CONHCH_3$ | 202-6 |
| 115 | " | " | " | " | $-CON(CH_3)_2$ | Öl |
| 116 | " | " | " | " | $-CONHCH(CH_3)_2$ | 90-5 |
| 117 | " | $C_2H_5$ | Cl | " | $-COO-CH(CH_3)_2$ | 59-63 |
| 118 | " | " | " | " | $-COO-CH(CF_3)_2$ | Öl |
| 119 | " | " | " | " | $-COOCH_2-CH=CH_2$ | Öl |
| 120 | " | " | " | " | $-CONHC_2H_5$ | Öl |

Fortsetzung Tabelle 1

| Beisp.-Nr | $R^1$ | $R^2$ | $R^3$ | $R^4$ | Y | Fp/Sdp(°C) |
|---|---|---|---|---|---|---|
| 121 | $CH_3$ | $C_2H_4$ | Cl | H | $-CONHCH(CH_3)_2$ | 171 |
| 122 | " | " | " | " | $-CONHCH_2C(CH_3)_3$ | 165-9 |
| 123 | $C_2H_5$ | " | " | " | -COOLi | 278-83 |
| 124 | " | " | " | " | $-COOCH(CF_3)_2$ | Oel |
| 125 | " | " | " | " | $-COOCH_2CF_2CHFCF_3$ | Oel |
| 126 | " | " | " | " | $-CON(CH_3)CH_2CH(OCH_3)_2$ | Oel |
| 127 | " | " | " | " | $-CONHCH_2CH(OC_2H_5)_2$ | 158-60 |
| 128 | " | " | " | " | $-CONHC_3H_7$ | 176-80 |
| 129 | " | " | " | " | $-CON(CH(CH_3)_2)_2$ | Oel |
| 130 | " | " | " | " | $-CONHCH_2OC_2H_5$ | 85-88 |
| 131 | " | " | " | " | $-CONHCH_2OCH_3$ | |
| 132 | " | " | " | " | $-CONHCH_2OC_3H_7$ | |
| 133 | " | " | Br | " | -COOK | >250 |
| 134 | " | " | " | " | $-CONH_2$ | 211-3 |
| 135 | " | " | " | " | $-CONHCH_2CH(OCH_3)_2$ | 98-101 |
| 136 | " | " | Cl | 5-Cl | -COOK | >270 |
| 137 | " | " | " | " | $-COOCH(CH_3)_2$ | 80-4 |
| 138 | " | " | " | " | $-COOC_3H_7$ | Oel |
| 139 | " | " | " | " | $-COSC_4H_9$ | Oel |
| 140 | " | " | " | " | $-CONHCH_3$ | 190-5 |
| 141 | " | " | " | " | $-CON(C_2H_5)_2$ | 92-7 |
| 142 | " | " | " | $4-CH_3$ | -COONa | >300 |
| 143 | " | " | " | " | -COOK | 214-6 |
| 144 | " | " | " | " | $-CO\overset{\ominus}{O}H\overset{\oplus}{N}(CH_2CH_2OH)_3$ | 120-2 |
| 145 | $C_2H_5$ | $C_2H_5$ | Cl | $4-CH_3$ | $-COOCH(CH_3)_2$ | 92-5 |
| 146 | " | " | " | " | $-COOC_4H_9$ | Oel |
| 147 | " | " | " | " | $-COOCH_2CF_3$ | Oel |
| 148 | " | " | " | " | $-COOCH_2-CH=CH_2$ | Oel |
| 149 | " | " | " | " | $-CONHCH_3$ | 186-8 |
| 150 | " | " | " | " | $-CON(CH_3)_2$ | Oel |

Fortsetzung Tabelle 1

| Beisp.-Nr | $R^1$ | $R^2$ | $R^3$ | $R^4$ | Y | Fp/Sdp (°C) |
|---|---|---|---|---|---|---|
| 151 | $C_2H_5$ | $C_2H_5$ | Cl | 4-$CH_3$ | $-CONHC_2H_5$ | 146-7 |
| 152 | " | " | " | " | $-CONHCH(CH_3)_2$ | 146-8 |
| 153 | " | " | " | " | $-CONHCH_2-CH(CH_3)_2$ | 192-4 |
| 154 | " | " | " | " | $-CONH(CH_2)_3OCH_3$ | 122-4 |
| 155 | " | " | " | " | $-CONHCH_2CH(OC_2H_5)_2$ | 139-40 |
| 156 | " | $CH_3$ | " | H | -COOK | Harz |
| 157 | " | " | " | " | $-COOCH_2CF_3$ | Oel |
| 158 | " | " | " | " | $-COOC_4H_9$ | Oel |
| 159 | " | " | " | " | $-COOCH_2CH_2OCH_3$ | Oel |
| 160 | " | " | " | " | $-CONHCH_3$ | Harz |
| 161 | " | " | " | " | $-CONHCH_2CH(CH_3)_2$ | 150-9 |
| 162 | " | " | " | " | $-CONHCH_2CH(OCH_3)_2$ | 115-9 |

## Biologische Beispiele

### 1. Wuchshemmung an Getreide

In Schalenversuchen im Gewächshaus wurden junge Getreidepflanzen (Weizen, Gerste, Roggen)im 3-Blattstadium mit erfindungsgemäßen Verbindungen sowie einer Vergleichsverbindung aus DE-A 32 17 094 in verschiedenen Wirkstoffkonzentrationen (kg/ ha) tropfnaß gespritzt.

Nachdem die unbehandelten Kontrollpflanzen eine Wuchshöne von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet. Die Wuchshemmung wurde als prozentualer Wert ermittelt, wobei 100 % den Stillstand des Wachstums und 0 % ein Wachstum entsprechend den unbehandelten Kontrollpflanzen bedeuten. Es zeigte sich, daß die Verbindungen sehr gute wachstumsregulierende Eigenschaften besitzen.

Die Ergebnisse sind in der nachfolgenden Tabelle 2 zusammengestellt.

T a b e l l e  2

| Verbind. nach Bsp.Nr. | Dosis (kg a.i./ha) | Wuchshemmung (%) Weizen | Gerste | Roggen | Phytotox. Wirkung |
|---|---|---|---|---|---|
| 39 | 2.5 | 27 | 35 | 27 | keine Schäden |
| | 1.25 | 22 | 27 | 25 | |
| | 0.62 | 18 | 19 | 18 | |
| 40 | 2.5 | 29 | 35 | 27 | keine Schäden |
| | 1.25 | 23 | 26 | 24 | |
| | 0.62 | 18 | 19 | 18 | |
| 51 | 2.5 | 25 | 36 | 18 | keine Schäden |
| | 1.25 | 10 | 31 | 11 | |
| | 0.62 | 5 | 23 | 5 | |
| 54 | 2.5 | 21 | 25 | 19 | keine Schäden |
| | 1.25 | 14 | 17 | 16 | |
| | 0.62 | 7 | 11 | 10 | |
| 64 | 2.5 | 19 | 37 | 19 | keine Schäden |
| | 1.25 | 16 | 30 | 10 | |
| | 0.62 | 11 | 24 | 4 | |
| 77 | 2.5 | 26 | 34 | 27 | keine Schäden |
| | 1.25 | 24 | 29 | 20 | |
| | 0.62 | 19 | 23 | 18 | |
| 81 | 2.5 | 28 | 36 | 27 | keine Schäden |
| | 1.25 | 24 | 28 | 24 | |
| | 0.62 | 19 | 20 | 19 | |
| 91 | 2.5 | 25 | 32 | 28 | keine Schäden |
| | 1.25 | 17 | 28 | 25 | |
| | 0.62 | 11 | 19 | 17 | |
| 127 | 2.5 | 21 | 26 | 19 | keine Schäden |
| | 1.25 | 13 | 19 | 15 | |
| | 0.62 | 7 | 12 | 7 | |

Fortsetzung Tabelle 2

| Verbind nach Bsp.Nr. | Dosis (kg a.i./ha) | Wuchshemmung (%) Weizen | Gerste | Roggen | Phytotox. Wirkung |
|---|---|---|---|---|---|
| 130 | 2.5 | 22 | 29 | 10 | keine |
| | 1.25 | 15 | 24 | 14 | Schäden |
| | 0.62 | 9 | 16 | 8 | |
| 133 | 2.5 | 28 | 35 | 26 | keine |
| | 1.25 | 22 | 26 | 21 | Schäden |
| | 0.62 | 18 | 21 | 17 | |
| 147 | 2.5 | 26 | 33 | 21 | keine |
| | 1.25 | 18 | 24 | 15 | Schäden |
| | 0.62 | 12 | 11 | 9 | |

2. Wuchshemmung in Wasserreis

Reispflanzen wurden angezogen und im Stadium der maximalen Bestockung mit den erfindungsgemäßen Verbindungen sowie mit einer Vergleichsverbindung aus DE-A 32 17 094 behandelt. Die Substanzen wurden sowohl durch Spritzung appliziert als direkt auch in das Wasser gegeben.

Drei Wochen nach Behandlung wurden bei allen Pflanzen der Zuwachs gemessen und die Wuchshemmung in % des Zuwachses der Kontrollpflanzen berechnet. Es wurde außerdem auf eine mögliche phytotoxische Wirkung der Verbindung geachtet.

Die Ergebnisse sind in der nachfolgenden Tabelle zusammengefaßt.

Tabelle 3

| Verbind nach Bsp.Nr. | Dosis (kg/ha) | Wuchshemmung (%) | Phytotox. Wirkung |
|---|---|---|---|
| 39 | 2.5 | 43 | keine |
| | 1.25 | 41 | Schäden |
| | 0.62 | 33 | |
| 40 | 2.5 | 44 | keine |
| | 1.25 | 39 | Schäden |
| | 0.62 | 31 | |
| 51 | 2.5 | 30 | keine |
| | 1.25 | 23 | Schäden |
| | 0.62 | 15 | |
| 54 | 2.5 | 32 | keine |
| | 1.25 | 25 | Schäden |
| | 0.62 | 17 | |
| 64 | 2.5 | 31 | keine |
| | 1.25 | 23 | Schäden |
| | 0.62 | 17 | |
| 77 | 2.5 | 40 | keine |
| | 1.25 | 35 | Schäden |
| | 0.62 | 29 | |
| 81 | 2.5 | 46 | keine |
| | 1.25 | 41 | Schäden |
| | 0.62 | 35 | |
| 91 | 2.5 | 42 | keine |
| | 1.25 | 37 | Schäden |
| | 0.62 | 29 | |
| 127 | 2.5 | 31 | keine |
| | 1.25 | 24 | Schäden |
| | 0.62 | 17 | |
| 130 | 2.5 | 36 | keine |
| | 1.25 | 25 | Schäden |
| | 0.62 | 19 | |
| 147 | 2.5 | 33 | keine |
| | 1.25 | 25 | Schäden |
| | 0.62 | 19 | |

**Ansprüche**

1) Verbindungen der Formel I

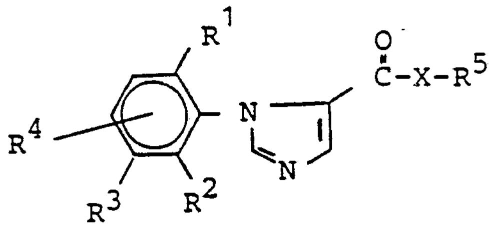

(I),

worin
$R^1$ und $R^2$ unabhängig voneinander ($C_1$-$C_4$)-Alkyl;
$R^3$ Halogen,
$R^4$ Wasserstoff, Cl, Br oder Methyl,
X O, S oder N-$R^5$
$R^5$ Wasserstoff, ($C_2$-$C_6$)-Alkenyl, ($C_1$-$C_6$)-Alkyl, wobei die Alkylgruppe bis zu zweifach durch ($C_1$-$C_6$)-Alkoxy, ($C_1$-$C_3$)-Dialkylamino oder bis zu sechsfach durch Halogen substituiert sein kann,
sowie deren für landwirtschaftliche Zwecke verträglichen Salze und Quarternisierungsprodukte.

2) Verfahren zur Herstellung der Verbindungen der Formel I und ihrer Salze oder Quaternisierungsprodukte, dadurch gekennzeichnet, daß man

a) einen Bisformylester der Formeln (IIa) oder (IIb)

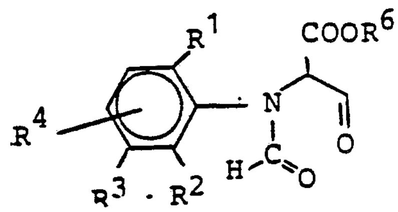

(IIa)

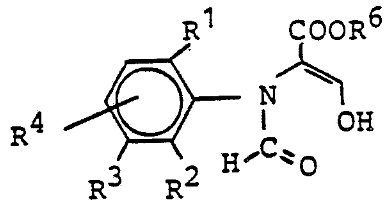

(IIb)

wobei $R^6$ = ($C_1$-$C_{12}$)-Alkyl bedeutet,
in Gegenwart von Ammoniak oder einer Ammoniak freisetzenden Verbindung erhitzt, oder

b) ein Aminophenylderivat der Formel III

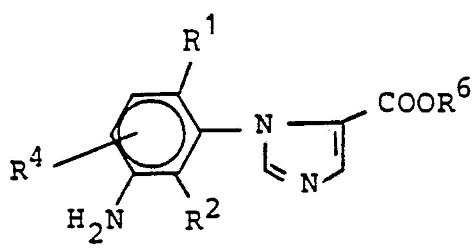

(III)

durch eine Sandmeyer-Reaktion in die Halogenverbindungen überführt, oder

c) für den Fall daß $R^3$ für Chlor oder Brom steht, ein Phenylimidazolderivat der Formel IV

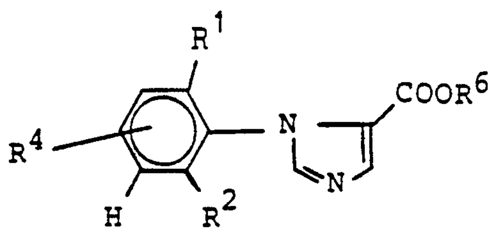

(IV)

am Phenylring bromiert oder chloriert oder die unter a), b) oder c) erhaltenen Verbindungen derivatisiert.

3) Pflanzenwachstumsregulierende Mittel, dadurch gekennzeichnet, daß sie eine wirksame Menge einer Verbindung der allgemeinen Formel (I) von Anspruch 1 oder deren Salze und übliche Trägerstoffe enthalten.

4) Verwendung von Verbindungen der allgemeinen Formel (I) von Anspruch 1 oder deren Salze als Wachstumsregulatoren für Pflanzen.

5) Verfahren zur Wachstumsregulierung von Pflanzen, dadurch gekennzeichnet, daß man auf die Pflanzen oder die Anbauflächen eine wirksame Menge einer Verbindung der allgemeinen Formel (I) von Anspruch 1 oder deren Salze appliziert.

Patentansprüche für den folgenden Vertragsstaat: Spanien:

1. Verfahren zur Herstellung von Verbindungen der Formel I sowie ihrer Salze und Quaternisierungsprodukte

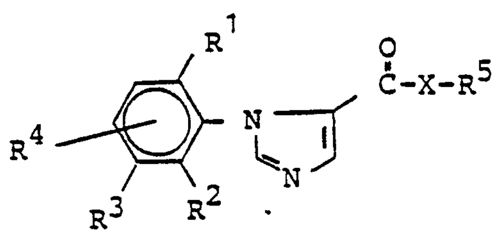

(I),

worin
$R^1$ und $R^2$ unabhängig voneinander ($C_1$-$C_4$)-Alkyl;
$R^3$ Halogen,
$R^4$ Wasserstoff, Cl, Br oder Methyl,
X O, S oder N-$R^5$
$R^5$ Wasserstoff, ($C_2$-$C_6$)-Alkenyl, ($C_1$-$C_6$)-Alkyl, wobei die Alkylgruppe bis zu zweifach durch ($C_1$-$C_6$)-Alkoxy, ($C_1$-$C_3$)-Dialkylamino oder bis zu sechsfach durch Halogen substituiert sein kann,
dadurch gekennzeichnet, daß man

a) einen Bisformylester der Formeln (IIa) oder (IIb)

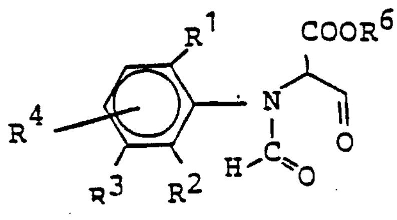

(IIa)

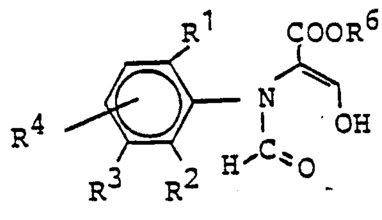

(IIb)

wobei $R^6$ = ($C_1$-$C_{12}$)-Alkyl bedeutet,
in Gegenwart von Ammoniak oder einer Ammoniak freisetzenden Verbindung erhitzt, oder

b) ein Aminophenylderivat der Formel III

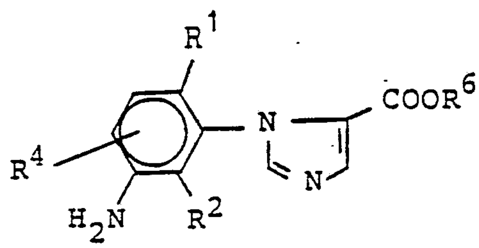

(III)

durch eine Sandmeyer-Reaktion in die Halogenverbindungen überführt, oder

c) für den Fall daß $R^3$ für Chlor oder Brom steht, ein Phenylimidazolderivat der Formel IV

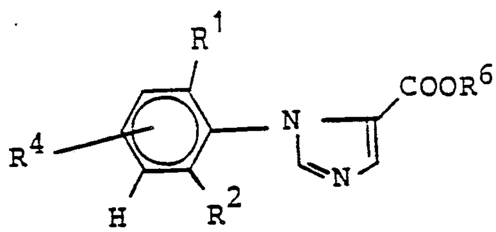

(IV)

am Phenylring bromiert oder chloriert oder die unter a), b) oder c) erhaltenen Verbindungen derivatisiert.

2) Pflanzenwachstumsregulierende Mittel, dadurch gekennzeichnet, daß sie eine wirksame Menge einer Verbindung der allgemeinen Formel (I) von Anspruch 1 oder deren Salze und übliche Trägerstoffe enthalten.

3) Verwendung von Verbindungen der allgemeinen Formel (I) von Anspruch 1 oder deren Salze als Wachstumsregulatoren für Pflanzen.

4) Verfahren zur Wachstumsregulierung von Pflanzen, dadurch gekennzeichnet, daß man auf die Pflanzen oder die Anbauflächen eine wirksame Menge einer Verbindung der allgemeinen Formel (I) von Anspruch 1 oder deren Salze appliziert.

Europäisches Patentamt

# EUROPÄISCHER RECHERCHENBERICHT

Nummer der Anmeldung

EP 87 11 2287

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 137 868 (HOECHST)<br>* Seiten 1-10; insbesondere Seite 2, Zeilen 29,30 *<br>--- | 1-5 | C 07 D 233/90<br>A 01 N 43/50 |
| D,X | DE-A-3 217 094 (HOECHST)<br>* Seiten 4-13; insbesondere Seite 5, Zeilen 29-30 *<br>--- | 1-5 | |
| Y | EP-A-0 185 961 (HOECHST)<br>* Seite 2, insbesondere Zeile 33; Seite 7, Zeile 36; Seiten 12-15 *<br>----- | 1,3-5 | |

RECHERCHIERTE SACHGEBIETE (Int. Cl.4)

C 07 D 233/00
A 01 N 43/50

Der vorliegende Recherchenbericht wurde für alle Patentansprüche erstellt

| Recherchenort | Abschlußdatum der Recherche | Prüfer |
|---|---|---|
| DEN HAAG | 04-12-1987 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)